# EUROPEAN PATENT APPLICATION

(11) **EP 4 818 201 A1**
(43) Date of publication of application: **07.10.2026**
(21) Application number: 24898053.4
(22) Date of filing: 25.11.2024
(51) Int. Cl.: C07K 14/72, C12N 15/86

(54) **FUSION PROTEIN ACTIVATED BY BLUE LIGHT AND USE THEREOF**

(30) Priority: 28.11.2023 KR 20230168491
(71) Applicant: AJOU UNIVERSITY INDUSTRY-ACADEMIC COOPERATION FOUNDATION, Gyeonggi-do 16499 (KR)
(72) Inventor: KIM, Seokhwi, Suwon-si, Gyeonggi-do 16499 (KR); LEE, Dakeun, Suwon-si, Gyeonggi-do 16499 (KR); WOO, Kyoungjin, Suwon-si, Gyeonggi-do 16499 (KR)
(74) Representative: Dolphin, Kirsty Mairi
(86) International application number: PCT/KR2024/018754
(87) International publication number: WO 2025/116459

(57) **Abstract**

The present invention relates to a fusion protein optogenetically activated by blue light, and a use thereof, wherein the fusion protein comprises: an extracellular domain of rhodopsin; a transmembrane domain of rhodopsin; and an intracellular domain of a G-protein coupled estrogen receptor (GPER) bound to the transmembrane domain. Specifically, the invention relates to a fusion protein that is activated by blue light, phosphorylates extracellular signal-regulated kinases (EPKs), inhibits apoptosis, and induces programmed necrosis.

## Description

### Technical Field

The present invention relates to a fusion protein activated by blue light and a use thereof, and more particularly, to a fusion protein that is optogenetically activated by light irradiation by combining an extracellular domain and a transmembrane domain of rhodopsin with an intracellular domain of a G-protein coupled estrogen receptor (GPER), and a use thereof.

### Related Art

Estrogen receptors are receptors belonging to the nuclear receptor superfamily having estrogen as a ligand, and are classified into two types: nuclear or intracellular receptors and a G-protein coupled estrogen receptor (GPER). Among them, GPER is a seven-transmembrane G-protein coupled receptor (GPCR) in which 17-β estradiol (E2), which is a type of steroid that is a sex hormone, acts as a receptor. GPER is mainly expressed in tissues such as reproductive organs, brain, and heart, and is known to be involved in cell growth, differentiation, proliferation, suppression of inflammation, angiogenesis, immunoregulation, neurotransmission, and the like.

GPCRs have a structure of seven transmembrane helices connected by three extracellular loops and three intracellular loops. The N-terminus, transmembrane domains 1-7, and extracellular loops 1-3 are generally associated with ligand binding and receptor activation, whereas intracellular loops 1-3 and the C-terminus contain residues that induce downstream signal transduction. GPCRs transmit signals through heterotrimeric G proteins composed of a Ga subunit of four major groups (Gas, Gai, Gaq, and Ga12) and a pair of b and r subunits.

When GPER is activated, it regulates estrogen signal transduction within cells and is involved in various physiological processes. Recently, it has been revealed that activation of GPER serves to inhibit the growth of cancer cells and to render tumors more immunogenic.

Rhodopsin is a visual receptor present in retinal rod cells, capable of absorbing visible light, and is a membrane-spanning receptor passing through the membrane seven times as a type of G protein-coupled receptor (GPCR). Rhodopsin is composed of opsin, which is a membrane protein fixed to the rod cell receptor membrane, and retinal, which functions to absorb light.

Optogenetics refers to a biological technique for controlling or monitoring the activity of cells and tissues by expressing ion channels whose opening and closing are regulated by light in cells or organisms, wherein the term means achieving genetic modification through light (opto). Various studies utilizing optogenetics technology have been conducted, however, studies applying optogenetics technology to estrogen receptors are still insufficient.

As conventional methods for activating GPER, methods of treating ligands G1 or E2 have been used. However, activation by ligand binding has disadvantages in that a temporal delay occurs because the ligand binds to the receptor by diffusion after treatment, and spatial precision such as activation at an individual cell level cannot be achieved.

Under such background, the present inventors first completed the present invention by confirming that activity is induced by blue light when a fusion protein, OptoGPER, is prepared by molecular biologically fusing an extracellular domain and a transmembrane domain of rhodopsin with an intracellular domain of GPER.

### [Prior Art Documents]

### [Patent Documents]

Korean Patent Publication No. 10-2023-0052831 (2023.04.20)
Korean Patent Publication No. 10-2023-0126401 (2023.08.30)

### [Non-Patent Document]

Natale CAet al., Cell Mol Gastroenterol Hepatol. 2020;10(4):868-880.e1.

### SUMMARY OF THE INVENTION

### TECHNICAL PROBLEM

An object of the present invention is to provide a fusion protein optogenetically activated by blue light, comprising: an extracellular domain of rhodopsin; a transmembrane domain of rhodopsin; and an intracellular domain of a G-protein coupled estrogen receptor (GPER) bound to the transmembrane domain.

Another object of the present invention is to provide a polynucleotide encoding the fusion protein.

Still another object of the present invention is to provide a recombinant vector comprising the polynucleotide.

Still another object of the present invention is to provide a method for preparing a fusion protein activated by blue light, comprising: treating a cell with the vector; and irradiating the cell with blue light to induce photoactivation.

Still another object of the present invention is to provide a composition for regulating the activity of the fusion protein by blue light irradiation, comprising the fusion protein or a polynucleotide encoding the same.

### TECHNICAL SOLUTION

In order to achieve the above object, the present invention provides a fusion protein optogenetically activated by blue light, comprising: an extracellular domain of rhodopsin; a transmembrane domain of rhodopsin; and an intracellular domain of a G-protein coupled estrogen receptor (GPER) bound to the transmembrane domain.

The present invention also provides a polynucleotide encoding the fusion protein.

The present invention also provides a recombinant vector comprising the polynucleotide.

The present invention also provides a method for preparing a fusion protein activated by blue light, comprising: treating a cell with the vector; and irradiating the cell with blue light to induce photoactivation.

The present invention also provides a composition for regulating an activity of a fusion protein by blue light irradiation, comprising the fusion protein or a polynucleotide encoding the same.

### ADVANTAGEOUS EFFECTS

The fusion protein optogenetically activated by blue light according to the present invention, comprising: an extracellular domain of rhodopsin; a transmembrane domain of rhodopsin; and an intracellular domain of a G-protein coupled estrogen receptor (GPER) bound to the transmembrane domain, is the first GPER activated by optogenetics, and has effects of being activated by blue light, phosphorylating extracellular signal-regulated kinase (ERK), inhibiting apoptosis, and inducing programmed necroptosis. In addition, the fusion protein can implement various GPER activation patterns that are not possible through conventional ligand treatment, thereby inducing various responses of downstream signaling pathways, which may help interpret phenomena occurring under physiological or disease conditions. Accordingly, the fusion protein can be widely utilized for the prevention and treatment of estrogen receptor-dependent or estrogen receptor-mediated diseases, such as cancer and menopause.

### BRIEF DESCRIPTION OF THE DRAWINGS

FIG. 1 is a schematic diagram showing OptoGPER according to the present invention.
FIG. 2 is a schematic diagram showing viral treatment and photoinduction experiments for confirming activation of OptoGPER.
FIG. 3 is a bar graph showing qRT-PCR experimental results confirming induction of mRNA activation of c-FOS, PTGS2, CTGF, and EGR1 genes by OptoGPER and the G1 ligand.
FIG. 4 is a bar graph showing qRT-PCR experimental results confirming induction of mRNA expression of MMP2 and COL12A1 genes according to the activation pattern of OptoGPER.
FIG. 5 shows western blot experimental results confirming induction of downstream protein activation by OptoGPER and the G1 ligand.
FIG. 6 shows experimental results confirming phosphorylation of ERK according to activation of OptoGPER using an ERK-KTR sensor.
FIG. 7 is a schematic diagram showing a transcriptome profile analysis experiment according to the activation time of OptoGPER.
FIG. 8 shows changes in expression of a GPER gene set according to the activation time of OptoGPER.
FIG. 9 shows bulk-RNA seq clustering of OptoGPER.
FIG. 10 shows results of gene ontology analysis of the G1 gene set.
FIG. 11 shows changes in expression of genes at the intersection of cellular response to TNF and negative apoptosis GO terms.
FIG. 12 shows clustering according to optoGPER-induced expressed genes in the TCGA-CESE dataset.
FIG. 13 shows GO analysis results according to clusters of TCGA-CESE samples.
FIGS. 14A to 14C show experimental results of cell death according to simultaneous activation of OptoGPER and OptoFAS, represented by bar graphs and confocal imaging.
FIGS. 14D to 14F show experimental results of cell death according to simultaneous activation of OptoGPER and optoRIPK3, represented by bar graphs and confocal imaging.
FIGS. 14G to 14J show experimental results of cell death according to simultaneous activation of OptoGPER and OptoMLKL, represented by bar graphs and confocal imaging.
FIG. 15 shows a predictive model of binding between GPER and RIPK3 (receptor-interacting serine/threonine protein kinase 3).
FIG. 16 shows immunoprecipitation experimental results confirming GPER-RIPK3 binding through immunoprecipitation.
FIG. 17 shows results of analyzing types of cell death using a Caspase-3 biosensor.
FIG. 18 shows results confirming activation of downstream pathway factors according to OptoRIPK3 alone and co-activation with optoGPER through western blotting.
FIG. 19 shows results confirming DAMP secretion according to OptoRIPK3 alone and co-activation with optoGPER through a cytokine array.
FIG. 20 shows acceleration of HMGB1 release according to OptoRIPK3 alone and co-activation with optoGPER, represented by confocal imaging.
FIG. 21 shows an overview and results of RNA-seq analysis according to simultaneous activation of OptoRIPK3 and optoGPER.
FIG. 22 shows GO analysis results for DEGs upon simultaneous activation of GPER and RIPK3.
FIGS. 23a and 23b show GSEA analysis results upon simultaneous activation of GPER and RIPK3.

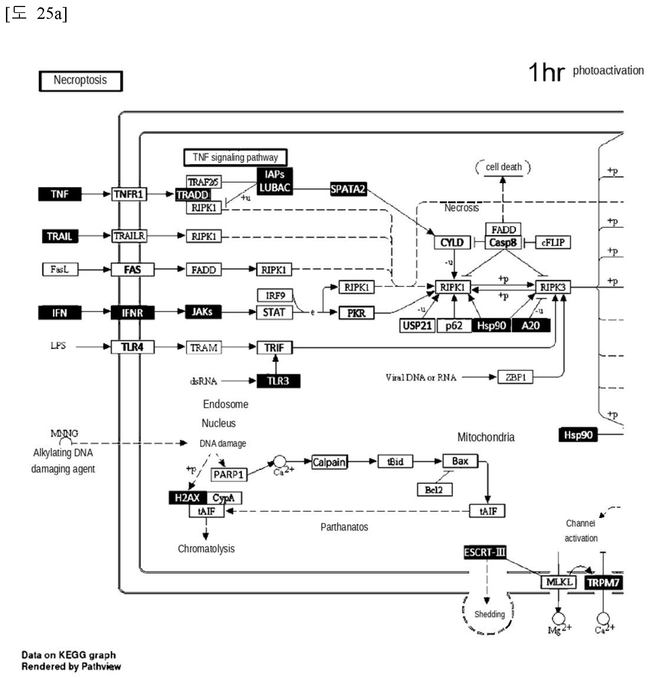

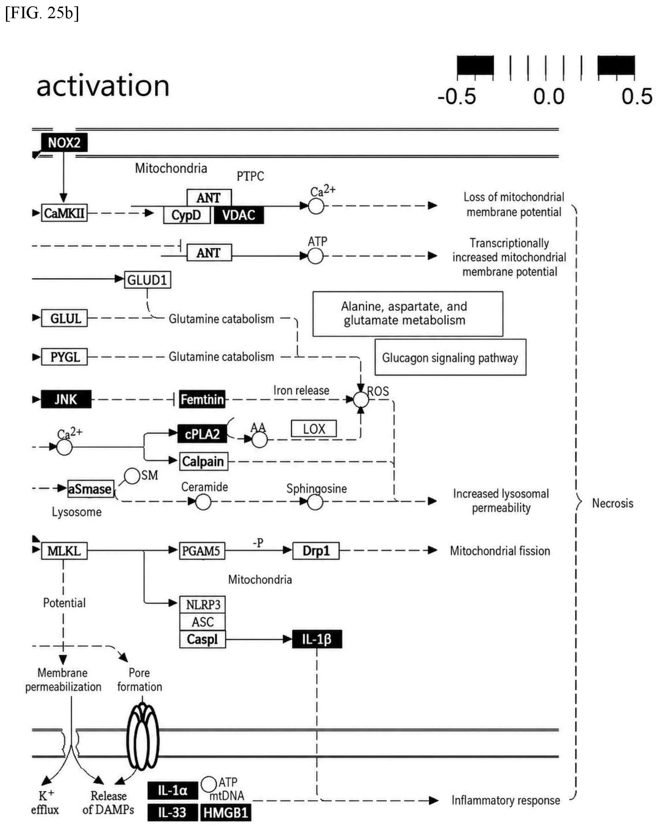

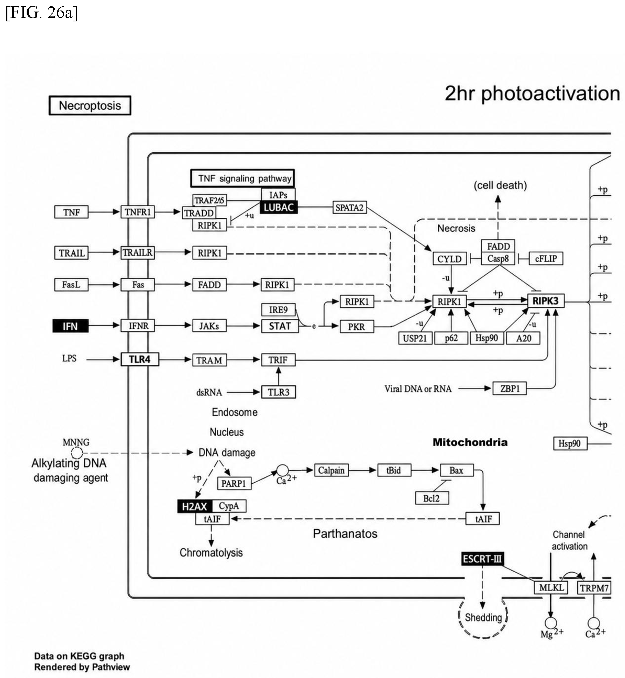

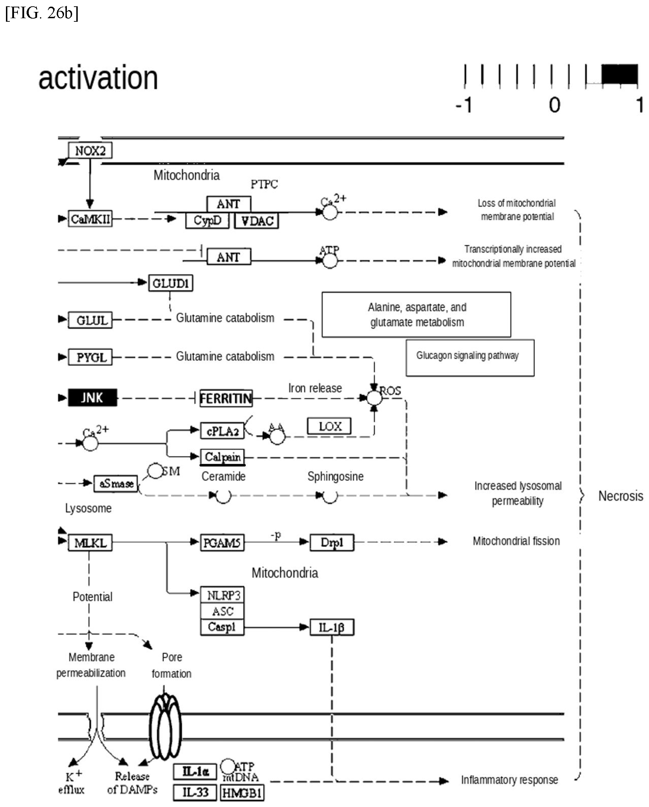

FIGS. 24a to 26b show time-dependent mapping of activated pathway factors in the necroptosis KEGG pathway upon simultaneous activation of OptoRIPK3 and optoGPER.

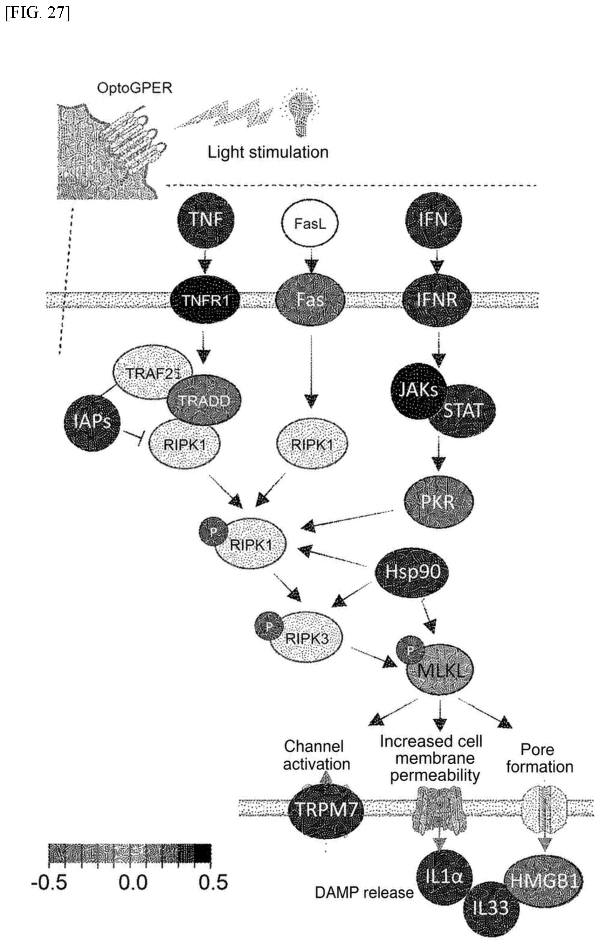

FIG. 27 is a schematic diagram showing induction of programmed necrosis pathway factor expression through OptoGPER.

### BEST MODE FOR CARRYING OUT THE INVENTION

Hereinafter, the present invention will be described in detail.

Unless otherwise defined, all technical and scientific terms used herein have the same meanings as commonly understood by those skilled in the art to which the present invention pertains. In general, the nomenclature used herein is well known and commonly used in the art.

As used herein, when any component or step is referred to as "comprising", this means that other components or steps are not excluded unless specifically stated to the contrary, and that other components or steps may be further included.

As used herein, the term "administration" means a method of placing a predetermined substance into a subject or by any method that results in at least partial localization to a desired site in the subject. Administration may be performed by methods known in the art.

As used herein, the term "polynucleotide" may have any three-dimensional structure and may perform any known or unknown function. The polynucleotide comprehensively includes DNA (gDNA and cDNA) and RNA molecules, and the nucleotide, which is the basic structural unit thereof, includes not only natural nucleotides but also analogues in which the sugar or base moiety is modified. The sequence of the polynucleotide may be modified, and such modifications include addition, deletion, non-conservative substitution, or conservative substitution of nucleotides.

As used herein, the term "vector" is understood as a nucleic acid means comprising a nucleotide sequence that can be introduced into a host cell and recombined and inserted into the genome of the host cell, or can self-replicate as an episome. The vector includes linear nucleic acids, plasmids, phagemids, cosmids, RNA vectors, viral vectors, and analogues thereof.

As used herein, the term "fusion protein" means a protein in which another protein is linked to the N-terminus or C-terminus of an original target protein sequence, or in which another amino acid sequence is added thereto.

The present invention provides a fusion protein optogenetically activated by blue light, comprising: an extracellular domain of rhodopsin; a transmembrane domain of rhodopsin; and an intracellular domain of a G-protein coupled estrogen receptor (GPER) bound to the transmembrane domain.

The domain refers to a protein domain, meaning a portion or site required for a protein to perform a specific function. Protein domains consist of amino acids of various lengths ranging from 25 to 500 amino acids, and are composed of combinations of secondary structures such as α-helices, β-sheets, β-turns, and random coils.

The extracellular domain refers to a portion in which a protein or other biomolecule interacts with the extracellular environment, and is mainly located on the cell surface.

The transmembrane domain refers to a portion located within the cell membrane and may be involved in biological processes such as transport of substances or signal transduction through the cell membrane.

The intracellular domain refers to a portion located inside the cell and may include proteins or other molecules related to intracellular signal transduction and intracellular material exchange.

The fusion protein may be encoded by the nucleotide sequence of SEQ ID NO: 1:

5'ATGAATGGGACCGAGGGTCCAAATTTTTACGTACCCTTTAGTAACAAGACTGGCG
TGGTGCGCAGTCCATTCGAAGCCCCACAGTACTACCTCGCAGAGCCGTGGCAATTCTCA
ATGCTGGCCGCTTATATGTTCCTTCTGATTATGCTGGGGTTTCCCATCAATTTTCTTACCC
TGTATGTGATCAGCTTCCGCGAGAAGATGACCATCCCCTTGAATTATATTCTTCTGAACC
TCGCGGTGGCAGATCTTTTCATGGTGTTCGGCGGGTTTACGACTACTCTGTATACGTCCC
TGCATGGTTATTTTGTGTTCGGGCCCACAGGCTGCAACTTGGAAGGCTTCTTCGCCACTC
TTGGCGGTGAGATCGCTCTTTGGAGCCTGGTCGTCCTGGCCATCGAGCGGTATGTGGTG
GTGGACCGCTACATCGCCCTGGCCAGGGCCATGCGCTGCAGCCTGTTCCGCACCAAGC
ACCACGCCCGGGCCATTATGGGTGTAGCCTTTACCTGGGTCATGGCTTTGGCCTGTGCT
GCTCCCCCCCTGGTGGGTTGGTCCCGCTATATTCCAGAAGGTATGCAGTGTTCTTGCGG
AATCGACTACTATACCCCGCACGAAGAGACAAACAACGAGTCCTTCGTCATATATATGT
TTGTAGTCCACTTTATCATCCCCTTGATTGTTATTTTTTTTTGCTATGGACTCATTGTCCGG
GTGCTGGTCAGGGCGCACCGGCACCGTGGGCTGCGGCCCCGGCGGCAGAAGGCGCGC
ATGGTCATCATCATGGTTATCGCCTTTCTCATTTGCTGGCTGCCTTACGCTGGAGTCGCG
TTTTACATCTTCACACATCAAGGTTCTGACTTCGGCCCAATCTTTATGACCATCCCTGCCT
TCTTCGCCAAGACCTCTGCCGTGTATAACCCCGTTATCTATATTATGATGAACAAGCAGT
TCCGGGAGACCTTCAGGGACAAGCTGAGGCTGTACATTGAGCAGAAAACAAATTTGCC
GGCCCTGAACCGCTTCTGTCACGCTGCCCTGAAGGCCGTCATTCCAGACAGCACCGAG
CAGTCGGATGTGAGGTTCAGCAGTGCCGTGACAGAAACTTCACAGGTTGCCCCCGCC 3'
(SEQ ID NO: 1).

The fusion protein may be activated by irradiation with blue light having a wavelength of 350 to 500 nm and an intensity of 500 to 1500 uW/cm² for 1 to 10 days, and preferably may be activated by irradiation with blue light having a wavelength of 400 to 500 nm and an intensity of 700 to 1300 uW/cm² for 1 to 7 days, but is not limited thereto.

In addition, the blue light may be irradiated for 0.1 to 10 hours, and preferably for 0.1 to 8 hours, but is not limited thereto.

The fusion protein may increase mRNA expression of one or more genes selected from the group consisting of c-FOS, PTGS2, CTGF, EGR1, MMP2, and COL12A1, the expression of which is increased by activated GPER, but is not limited thereto.

The fusion protein may further comprise a fluorescent protein. The fluorescent protein may be, for example, green fluorescent protein (GFP), enhanced green fluorescent protein (EGFP), yellow fluorescent protein (YFP), red fluorescent protein (RFP), orange fluorescent protein (OFP), cyan fluorescent protein (CFP), blue fluorescent protein (BFP), far-red fluorescent protein, or tetracysteine motif, but is not limited thereto.

The fusion protein may phosphorylate extracellular signal-regulated kinase (ERK).

The extracellular signal-regulated kinase is a type of mitogen-activated protein kinase (MAPK). MAPK is a type of serine/threonine protein kinase and plays a very important role in intracellular signal transduction systems in response to various external stimuli, and is involved in regulation of expression of many genes. MAPKs are classified into ERK1, ERK2, and SAPKs (stress-activated kinases). The SAPKs may also be referred to as JNKs (c-Jun N-terminal kinase).

The fusion protein may inhibit apoptosis and induce programmed necroptosis.

Apoptosis is a cell death pathway regulated by activation of cysteine proteases known as caspases, and may also be referred to as programmed cell death or apoptosis.

Programmed necroptosis involves RIPK1, RIPK3, and MLKL proteins, which are sequentially activated to form a large complex called a necrosome, and induce cell death through MLKL-mediated membrane rupture without protein degradation or oxidation processes. Recently, it has been confirmed that RIPK3 knockout in mice improves neuronal damage in acute pancreatitis, ischemia-reperfusion injury, systemic inflammatory response syndrome, terminal ileitis, retinal detachment, inflammatory bowel disease, atherosclerosis, and Gaucher disease compared to wild-type mice, thereby revealing that programmed necroptosis is associated with various immune diseases and diseases related to cellular tissue damage.

The programmed necroptosis may occur through binding with RIPK3, but is not limited thereto.

The present invention also provides a polynucleotide encoding the fusion protein.

The present invention also provides a recombinant vector comprising the polynucleotide.

The vector components may generally include one or more selected from the group consisting of a signal sequence, replication origin, one or more marker genes, enhancer elements, promoters, and transcription termination sequences, but are not limited thereto.

The vector may be one or more viruses selected from the group consisting of adeno-associated virus (AAV), adenovirus, retrovirus, lentivirus, modified vaccinia virus ankara (MVA), herpes simplex virus, and baculovirus, and preferably may be an adenovirus, but is not limited thereto.

The present invention also provides a method for preparing a fusion protein activated by blue light, comprising: treating a cell with the vector; and irradiating the cell with blue light to induce photoactivation.

The present invention also provides a composition for regulating an activity of a fusion protein by blue light irradiation, comprising the fusion protein or a polynucleotide encoding the same.

The composition may inhibit apoptosis and induce programmed necroptosis, but is not limited thereto.

The composition may further comprise a pharmaceutically acceptable carrier for administration of the composition. The pharmaceutically acceptable carrier may be any non-toxic substance suitable for delivery to a patient. Distilled water, alcohols, fats, waxes, and inert solids may be included as carriers. Pharmacologically acceptable adjuvants (buffers, dispersants) may also be included in the pharmaceutical composition.

Specifically, the pharmaceutical composition may be prepared as a parenteral formulation according to conventional methods known in the art depending on the route of administration, together with a pharmaceutically acceptable carrier in addition to the active ingredient. As used herein, "pharmaceutically acceptable" means that the applicable subject does not exhibit toxicity beyond an adaptable level while not inhibiting the activity of the active ingredient.

When the pharmaceutical composition is prepared as a parenteral formulation, it may be formulated in the form of injections, transdermal preparations, nasal inhalants, and suppositories according to methods known in the art together with an appropriate carrier. When formulated as an injection, sterile water, polyols such as ethanol, glycerol, or propylene glycol, or mixtures thereof may be used as suitable carriers, and preferably isotonic solutions such as Ringer's solution, PBS (phosphate buffered saline) containing triethanolamine, sterile water for injection, 5% dextrose may be used.

The compositions, methods, and uses of the present invention may be administered to a subject in need thereof in a sufficient amount or an effective amount. The term "effective amount" or "sufficient amount" refers to an amount that provides a benefit to the subject or provides an expected or desired result to the subject over any period of time, either alone or in combination with one or more other therapeutic compositions, protocols, or treatment regimens, in a single or multiple doses. It may be variously prescribed depending on factors such as formulation method, mode of administration, age, weight, sex, pathological condition, administration time, administration route, excretion rate, and response sensitivity of the patient.

Hereinafter, the present invention will be described in more detail through Examples. These Examples are provided only for more specifically describing the present invention, and it will be obvious to those skilled in the art that the scope of the present invention is not limited by these Examples according to the gist of the present invention.

### 1. Development of OptoGPER and Confirmation of Optogenetic Activation

### 1-1. Development of OutoGPER

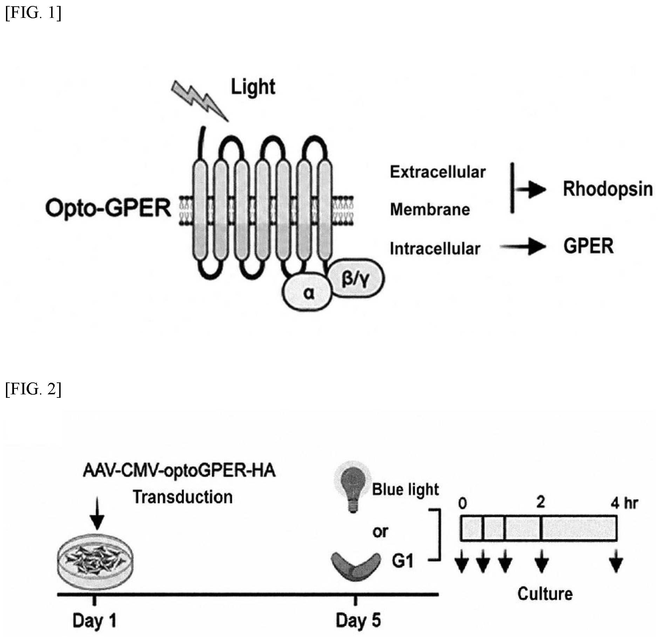

As shown in FIG. 1, optoGPER (SEQ ID NO: 1) was constructed based on the DNA sequence of rhodopsin, in which the intracellular loops and C-terminus were replaced with DNA nucleotide sequences constituting the intracellular domains of GPER:

5'ATGAATGGGACCGAGGGTCCAAATTTTTACGTACCCTTTAGTAACAAGACTGGCG
TGGTGCGCAGTCCATTCGAAGCCCCACAGTACTACCTCGCAGAGCCGTGGCAATTCTCA
ATGCTGGCCGCTTATATGTTCCTTCTGATTATGCTGGGGTTTCCCATCAATTTTCTTACCC
TGTATGTGATCAGCTTCCGCGAGAAGATGACCATCCCCTTGAATTATATTCTTCTGAACC
TCGCGGTGGCAGATCTTTTCATGGTGTTCGGCGGGTTTACGACTACTCTGTATACGTCCC
TGCATGGTTATTTTGTGTTCGGGCCCACAGGCTGCAACTTGGAAGGCTTCTTCGCCACTC
TTGGCGGTGAGATCGCTCTTTGGAGCCTGGTCGTCCTGGCCATCGAGCGGTATGTGGTG
GTGGACCGCTACATCGCCCTGGCCAGGGCCATGCGCTGCAGCCTGTTCCGCACCAAGC
ACCACGCCCGGGCCATTATGGGTGTAGCCTTTACCTGGGTCATGGCTTTGGCCTGTGCT
GCTCCCCCCCTGGTGGGTTGGTCCCGCTATATTCCAGAAGGTATGCAGTGTTCTTGCGG
AATCGACTACTATACCCCGCACGAAGAGACAAACAACGAGTCCTTCGTCATATATATGT
TTGTAGTCCACTTTATCATCCCCTTGATTGTTATTTTTTTTTGCTATGGACTCATTGTCCGG
GTGCTGGTCAGGGCGCACCGGCACCGTGGGCTGCGGCCCCGGCGGCAGAAGGCGCGC
ATGGTCATCATCATGGTTATCGCCTTTCTCATTTGCTGGCTGCCTTACGCTGGAGTCGCG
TTTTACATCTTCACACATCAAGGTTCTGACTTCGGCCCAATCTTTATGACCATCCCTGCCT
TCTTCGCCAAGACCTCTGCCGTGTATAACCCCGTTATCTATATTATGATGAACAAGCAGT
TCCGGGAGACCTTCAGGGACAAGCTGAGGCTGTACATTGAGCAGAAAACAAATTTGCC
GGCCCTGAACCGCTTCTGTCACGCTGCCCTGAAGGCCGTCATTCCAGACAGCACCGAG
CAGTCGGATGTGAGGTTCAGCAGTGCCGTGACAGAAACTTCACAGGTTGCCCCCGCC 3'
(SEQ ID NO: 1).

For visualization during cell imaging through microscopy, OptoGPER-EGFP and OptoGPER-miRFP were constructed by tagging EGFP (enhanced green fluorescent protein) and miRFP (monomeric infrared fluorescent protein) fluorescent proteins at the C-terminus of OptoGPER.

For this purpose, EGFP and miRFP were amplified by PCR from CMV-EGFP-N1 and CMV-miRFP670-N1 vectors, respectively, and NotI/XbaI restriction enzymes (New England Biolabs) were used. The amplified products were inserted downstream of the optoGPER sequence using the Gibson assembly cloning method (New England Biolabs). The primers used were as follows:

**[Table 1]**

| | Nucleotide sequence | SEQ ID NO. |
|---|---|---|
| Forward primer | 5' GGTTGCCCCCGCCGCGGCCGCCA TGGTGAGCAAGG3' | 2 |
| Reverse primer | 5'CGAACCGCGGGCCCTCTAGATT ACTTGTACAGCTCGTCCATGC 3' | 3 |

To construct OptoGPER in the form of an adeno-associated virus (AAV) for treatment of cells, an HA tag was inserted at the C-terminus of OptoGPER to construct OptoGPER-HA. Thereafter, DIO-optoFGFR1-HA was removed from the AAV-CMV-DIO-optoFGFR1-HA vector using XbaI/HindIII restriction enzymes (New England Biolabs), and replaced with PCR-amplified optoGPER-HA and EGFP, respectively, thereby constructing AAV-CMV-optoGPER-HA and AAV-CMV-EGFP.

**[Table 2]**

| | | Nucleotide sequence | SEQ ID NO. |
|---|---|---|---|
| Primer used for construction of OptoGPER-HA | Forward primer | 5' GTAAAGCTTGCCACCATGAATG GGACC 3' | 4 |
| | Reverse primer | 5'gtaACCGGTttaAGCATAATCTG GAACATCATATGGATAacctccGG CGGGG GCAACCTGTGAA 3' | 5 |
| Primer used for contruction of AAV-CMV-optoGPER-HA and AAV-CMV-EGFP | Forward primer | 5' GTATCTAGAGCCACCATGAATG GGACCGAGG3' | 6 |
| | Reverse primer | 5' GTAAAGCTTTTACTTGTACAGCT CGTCCATGCCG 3' | 7 |
| EGFP | Forward primer | 5' GTATCTAGAGCCACCATGGTGA GCAAGGGC3' | 8 |
| | Reverse primer | 5' GTAAAGCTTTTACTTGTACAGCT CGTCCATGCCG 3' | 9 |

### 1-2. Confirmation of Induction of mRNA Expression by Activation of OptoGPER

An experiment was conducted to confirm whether the constructed optoGPER could induce activation of downstream signaling pathways according to activation of GPER, thereby increasing mRNA expression of various factors known to exhibit induced expression. For the experiment, AAV-CMV-optoGPER-HA virus was treated into HeLa cells, and after 4 days of culture, photoactivation was induced using a blue LED. In addition, after treatment and culture with AAV-CMV-EGFP virus, the G1 ligand was treated for the same duration to induce ligand-mediated activation of GPER, and the results were compared with those of optoGPER (FIG. 2).

HeLa cells were maintained in DMEM (Gibco) supplemented with 10% fetal bovine serum (FBS), 1% penicillin and streptomycin, at 37°C in a humidified atmosphere containing 5% CO₂. For the experiment, 2×10⁵ cells were seeded into a 6-well plate, and one day later, AAV-CMV-optoGPER-EGFP and AAV-CMV-EGFP were transduced. After at least 24 hours, the medium was replaced with phenol red-free DMEM (Cat. No. LM 001-10, Welgene) supplemented with 10% fetal bovine serum stripped with dextran-coated charcoal (Sigma, C6241-5G), 1% penicillin and streptomycin, and maintained in a humidified atmosphere containing 5% CO₂ at 37°C. Four days after transduction, light stimulation was applied using 488 nm blue light at an intensity of 919.43 uW/cm² for 0, 0.25, 0.5, 1, 2, and 4 hours. G1 (Tocris, 3577) was treated at a concentration of 100 nM.

AAV was produced using a three-plasmid co-transfection system. Briefly, a transfer plasmid (including a transgene expression cassette), a packaging plasmid (pRC-DJ/8), and a helper plasmid (pHelper) were diluted in Opti-MEM (Cat. No. 31985-070, Gibco) at a ratio of 1:1:2. Polyethylenimine (PEI) was added to the solution to generate a DNA (ug)/PEI (ng) mixture at a ratio of 2.5:1. After incubation at room temperature for 15 minutes, the solution was distributed into 15 cm dishes in which HEK293T cells had grown to approximately 75-80% confluency. Four hours after transfection, the culture medium was completely replaced. At 60 to 72 hours after transfection, cells were harvested and centrifuged at 3300 g. Pellets were resuspended in 14 ml of lysis buffer (50 mM tris-Cl (pH 8.0), 150 mM NaCl, and 2 mM MgCl₂) at 4°C for 20 minutes and mixed with 10% sodium deoxycholate (final concentration, 0.5%) and benzonase (final concentration, 50 U/ml). Each lysate was incubated at 37°C for 30 minutes and subjected to 3 to 4 freeze-thaw cycles, followed by centrifugation at 12,000 g for 30 minutes at 4°C. The supernatant was loaded onto an iodixanol gradient layer and ultracentrifuged at 69,000 rpm for 1 hour at 4°C. The 40% iodixanol fraction was collected, washed four times with cold PBS using a 100,000 molecular weight cut-off Amicon tube, and concentrated by centrifugation at 3000 g to obtain a volume of 150 to 200 µl.

For transduction using AAV, the viral concentrate was added to plates and incubated at 37°C. After 24 hours, the medium was completely replaced with maintenance medium. A multiplicity of infection (MOI) of 5,000 to 40,000 was used for transduction.

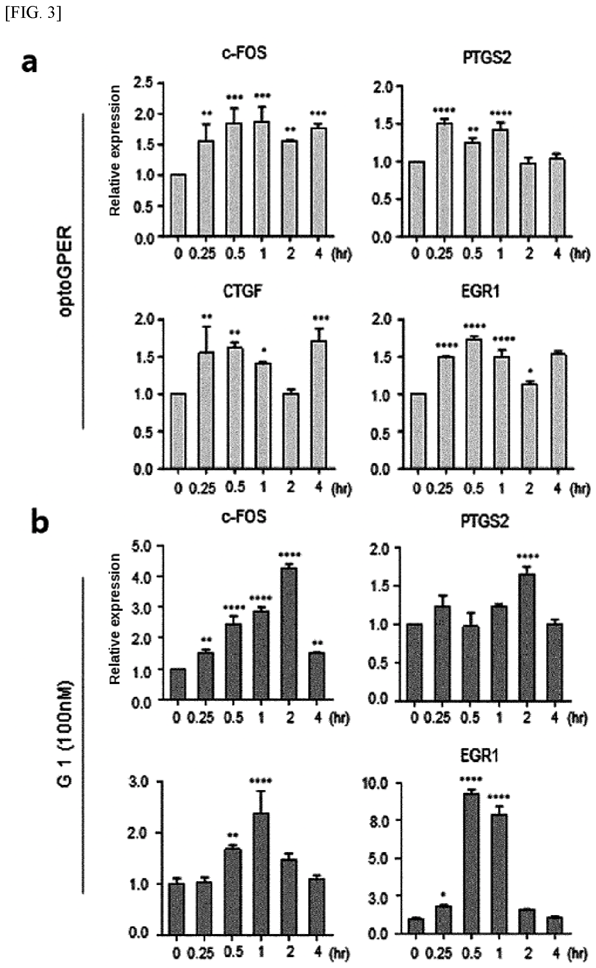

The mRNA expression levels of c-FOS, PTGS2, CTGF, and EGR1 genes, which are known to be increased by activated GPER, were confirmed by qRT-PCR. Although the expression patterns and levels of target genes over time differed between the group treated with 100 nM G1 and the group subjected to light stimulation of optoGPER, an overall increasing tendency was observed in both groups (FIG. 3).

Additionally, in order to analyze differences in induction of downstream signaling pathways of OptoGPER according to photoactivation patterns, NIH3T3 cells were transduced with OptoGPER using AAV, subjected either to continuous light stimulation or pulsed light stimulation (1 second light stimulation followed by 9 seconds rest), and expression levels of MMP2 and COL12A1 mRNA were confirmed by qRT-PCR (Table 3).

**[Table 3]**

| | | Nucleotide sequence | SEQ ID NO. |
|---|---|---|---|
| MMP2 | Reverse primer | 5' TATTCTGGTCAAGATCACCTGTCTG 3' | 10 |
| | Forward primer | 5' CTGAAGGACACACTAAAGAAGATG C 3' | 11 |
| COL12A 1 | Reverse primer | 5' TATCCAAGAACTGGAGAAGGTGAA G 3' | 12 |
| | Forward primer | 5' GCTGTTTATGAAGATGGAGATGGTG 3' | 13 |

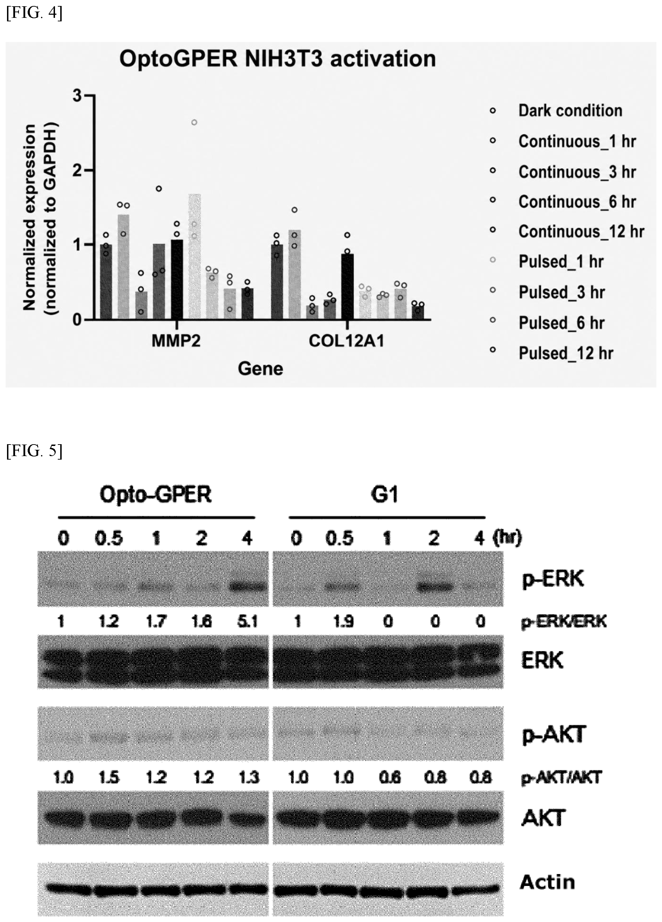

Continuous (Cons) and pulsed (Puls) light stimulation durations were varied as 1 hour, 3 hours, 6 hours, and 12 hours, while the light intensity was consistently maintained at 919.43 µW/cm². In the case of continuous light stimulation, the mRNA expression levels of MMP2 and COL12A1 decreased at 3 hours but recovered to normal levels at 12 hours. In contrast, pulsed light stimulation continuously suppressed the expression levels of both mRNAs from 1 to 3 hours after light stimulation (FIG. 4).

### 1-3. mRNA Extraction and qRT-PCR Experimental Method

RNA was extracted using an RNeasy Plus mini kit (Cat. No. 74134, QIAGEN) according to the manufacturer's instructions. cDNA was synthesized from the extracted RNA using the GoScript Reverse Transcription System (Cat. No. A5001, Promega) according to the manufacturer's instructions. qRT-PCR was performed using GoTaq qPCR Master Mix (Cat. No. A6002, Promega) according to the manufacturer's instructions, and carried out using a Thermal Cycler Dice Real-Time System III (Takara) with FrameStar 0.1 ml 96-well qPCR plates (Cat. No. NJ904, Takara). The mRNA expression levels of target genes were normalized to GAPDH mRNA expression levels and analyzed using the comparative Ct method (ΔΔCt). P-values were analyzed using Prism with one-way ANOVA. (* < 0.05, ** < 0.01, *** < 0.001, **** < 0.0001). Error bars were generated by calculating the standard deviation from the mean values of triplicate experiments for each dataset. The primers used were as follows:

**[Table 4]**

| | | Nucleotide sequence | SEQ ID NO. |
|---|---|---|---|
| EGR1 | Reverse primer | 5' Cgctaacccctctgtctactatt 3' | 14 |
| | Forward primer | 5' gactggtagctggtattgaggg 3' | 15 |
| CTGF (ccn2) | Reverse primer | 5' cccaaaatctccaagcctatcaa 3' | 16 |
| | Forward primer | 5' ccacagaatttagctcggtatgt 3' | 17 |
| PTGS2 | Reverse primer | 5' attcccttccttcgaaatgcaat 3' | 18 |
| | Forward primer | 5' cattgtaagttggtggactgtca 3' | 19 |
| CFOS | Reverse primer | 5' gttgtgaagaccatgacaggag 3' | 20 |
| | Forward primer | 5' ccatcttattcctttcccttcgg 3' | 21 |

### 1-4. Confirmation of Activation of Downstream Signaling Pathway Proteins by Activation of OutoGPER

Light stimulation was applied over time to a group of cervical cancer (HeLa) cells transduced with AAV-CMV-optoGPER-HA, and G1 at 100 nM was treated over time to a group of HeLa cells transduced with AAV-shortEF1a-EGFP, followed by confirmation of protein expression levels by western blotting. In the case of optoGPER, phosphorylated ERK (extracellular signal-regulated kinase) gradually increased from 30 minutes, decreased at 2 hours, and increased again at 4 hours. In the G1-treated group, an increase was also observed at 30 minutes, followed by a decrease at 1 hour and a subsequent increase again at 2 hours (FIG. 5).

### 1-5. Detailed Methods for Protein Extraction and Western Blot Experiment

Cell lysates were obtained using cell lysis buffer (Cell Signaling Technology, Massachusetts, USA) together with a protease/phosphatase inhibitor cocktail (Cell Signaling Technology). Protein concentrations were evaluated using a Bradford protein assay kit (Thermo Fisher Scientific, Massachusetts, USA). Subsequently, equal amounts of proteins were separated using 4-12% Bis-Tris gradient gels (Thermo Fisher Scientific) together with MOPS running buffer (Thermo Fisher Scientific) according to the manufacturer's instructions. The separated proteins were transferred onto polyvinylidene difluoride membranes. After blocking with skim milk or 5% BSA, the membranes were incubated with appropriate primary antibodies and HRP-conjugated secondary antibodies according to the instructions provided by the manufacturer. After washing, the membranes were imaged using chemiluminescence (iBright CL1000, Thermo Fisher Scientific). Information regarding the antibodies used is as follows: anti-pMLKL (Abcam), anti-MLKL (Cell Signaling Technology), anti-HA (Cell Signaling Technology), anti-AKT (Cell Signaling Technology), anti-ERK (Cell Signaling Technology), anti-pAKT(S473) (Cell Signaling Technology), anti-pERK(T202/Y204) (Cell Signaling Technology), and anti-beta actin (GeneTex).

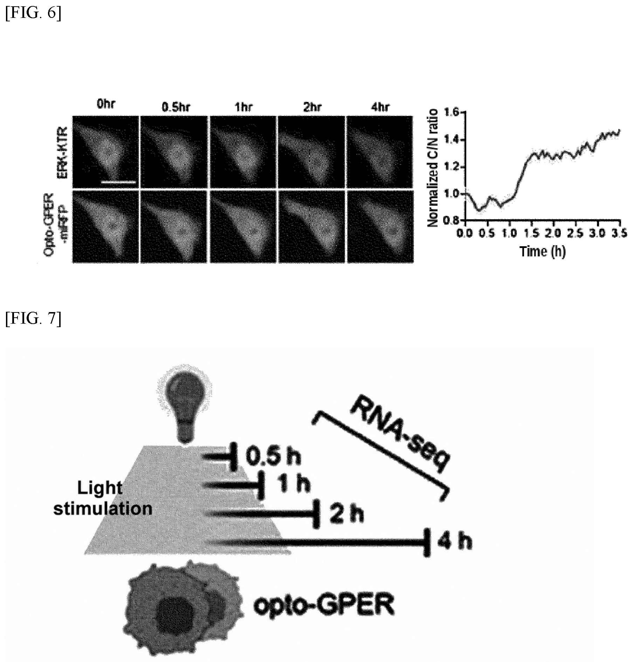

In addition, activation of pERK (phosphor-ERK) was confirmed through real-time confocal imaging. KTR (kinase translocation reporter) is a suitable method for studying kinase activity and localization, and is a genetically encoded fluorescent sensor that translocates to the cytoplasmic region upon phosphorylation. When ERK is phosphorylated and Elk1 (ETS transcription factor Elk-1), which is a substrate of extracellular signal-regulated kinase (ERK), binds to the ERK-KTR sensor present in the nucleus, the sensor translocates to the cytoplasm through phosphorylation-regulated nuclear localization signals (NLS) and nuclear export signals (NES), thereby allowing fluorescence to be detected in the cytoplasm. Upon activation of optoGPER, phosphorylation of ERK was confirmed using ERK-KTR, and it was confirmed that phosphorylation of ERK increased after 1 hour (FIG. 6).

### 2_{.} Analysis of Transcriptomic Changes According to the Activation Time of OptoGPER

### 2-1. Transcriptomic Changes According to Activation of OptoGPER in Cell Lines

Bulk RNA sequencing was used to analyze integrated changes in transcriptome profiles according to activation of OptoGPER. HeLa cells were seeded, transduced with AAV-CMV-optoGPER-EGFP on the following day, and after 4 days, light stimulation was applied at each time point, followed by rapid freezing in liquid nitrogen. Sequencing libraries were prepared from the prepared samples using TruSeq stranded RNA (Illumina), and NovaSeq6000 sequencing (100 bp paired-end, 6 Gb) was performed (Macrogen, FIG. 7).

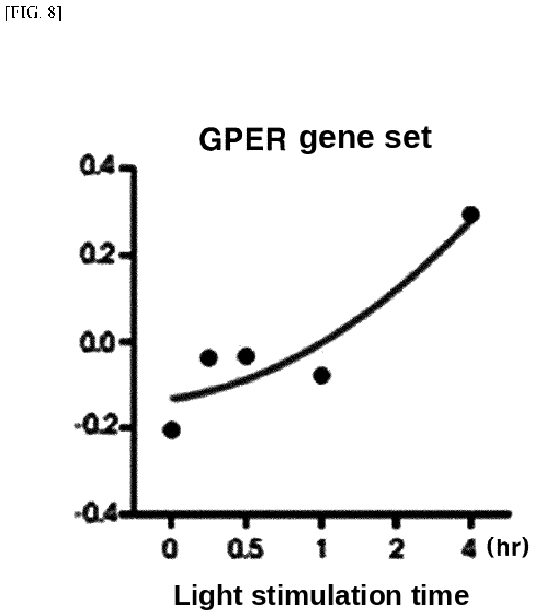

From the RNA-seq data obtained after activation of OptoGPER1, gene enrichment scores were quantified using a gene set (PTGS2, CCN1, EGR1, FOS, CCN2, and CCND1) known to be upregulated through GPER1 activation, and increases over time were confirmed (FIG. 8).

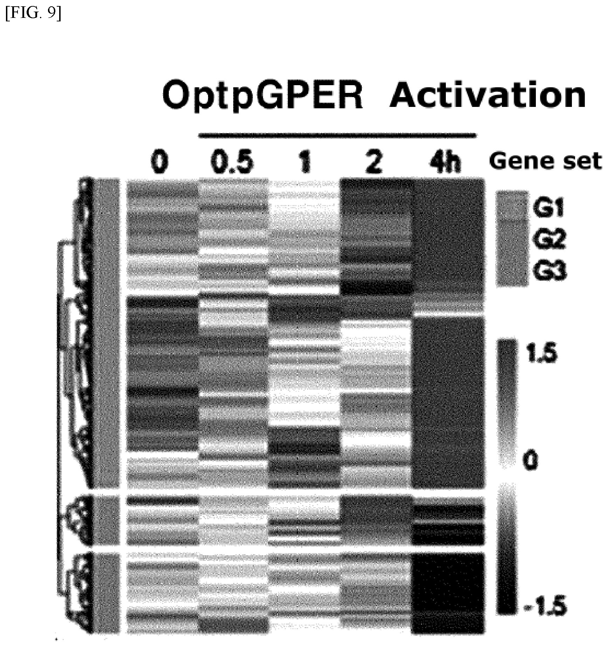

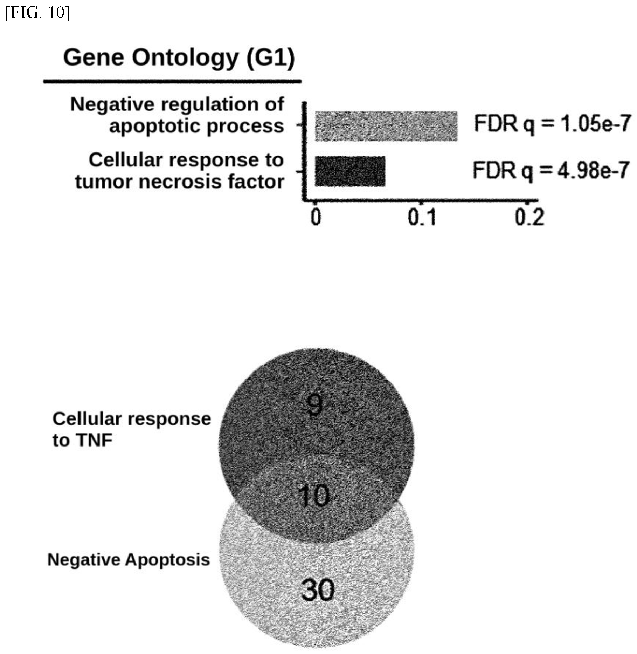

When the top 450 genes showing expression changes over time after activation of OptoGPER1 were subjected to Euclid clustering, they could be classified into a total of three gene sets. G1 is a group showing increases over time from 0 to 4 hours, G2 is a group showing increases from 0 to 2 hours but decreased expression at 4 hours, and G3 is a group showing continuous increases in expression followed by decreased expression at 4 hours (FIG. 9). Among these, gene ontology analysis performed on the genes constituting G1 revealed increased expression of apoptosis inhibitory genes (GO:0043066) and genes expressed in response to tumor necrosis factor (TNF) (GO:0071356) (FIG. 10).

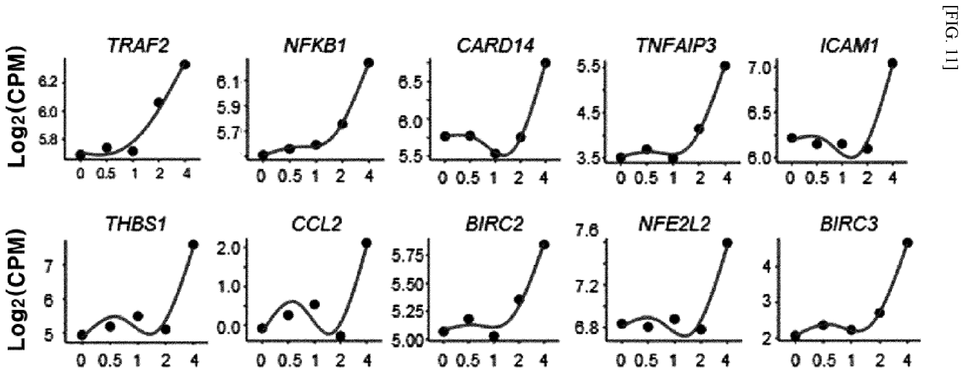

In addition, when expression changes according to optoGPER activation in 10 genes corresponding to the intersection of Cellular response to TNF (GO:0071356) and Negative Apoptosis (GO:0043066) in FIG. 9 were examined through RNA-seq data, increases over time were confirmed (FIG. 11). The red line represents a nonlinear expression pattern fitted to a natural spline.

### 2-2. Confirmation of Transcriptomic Changes According to GPER Activation in a Public Database

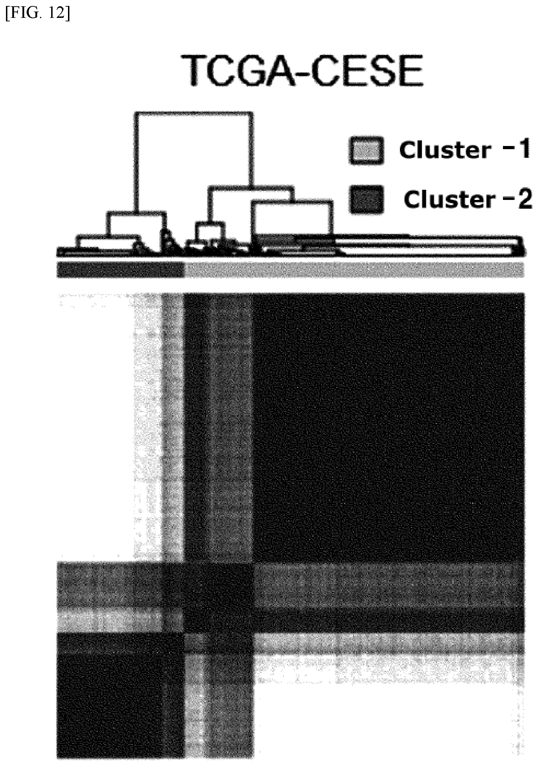

In order to confirm whether transcriptomic changes induced by activation of optoGPER in cell lines also occur in actual patient samples, analyses were conducted on 200 cases of Cervical Squamous Cell Carcinoma and Endocervical Adenocarcinoma (CESC) from the TCGA database. Using the top 450 genes expressed upon activation of optoGPER in HeLa cells, a heatmap generated through the Euclidean distance method and k-means algorithm showed that the overall cases were largely divided into two clusters (FIG. 12).

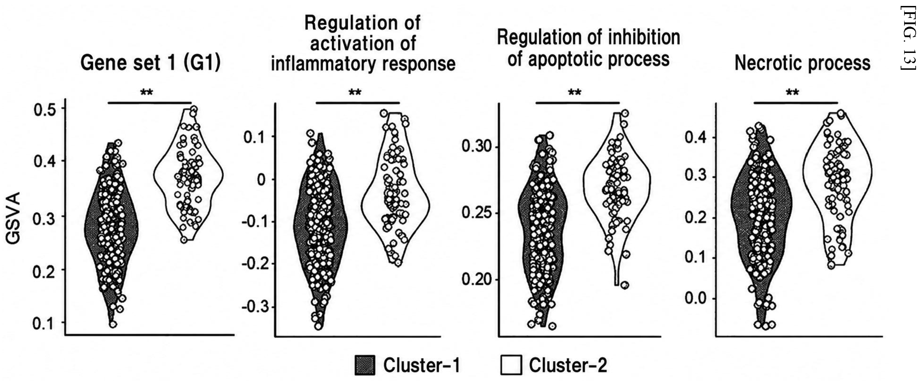

Among these, Cluster-2 showed statistically significantly increased expression of the G1 gene set identified in FIG. 8 compared to Cluster-1, from which it can be inferred that activation of GPER occurred in tumor samples belonging to the corresponding group. In addition, for the GO terms "positive regulation of inflammatory response" (GO:0050729), "negative regulation of apoptotic process" (GO:0043066), and "necroptotic process" (GO:0070266), samples included in Cluster-2 showed statistically significant increases in expression, which are results identical or similar to the GO categories increased in G1 shown in FIG. 9 (FIG. 13). Statistical differences were examined using Student's t-test (**P<0.05).

### 3. Analysis of the Effects of GPER on Cell Death Pathways

### 3-1. Analysis of the Effects of GPER Activation According to Types of Cell Death

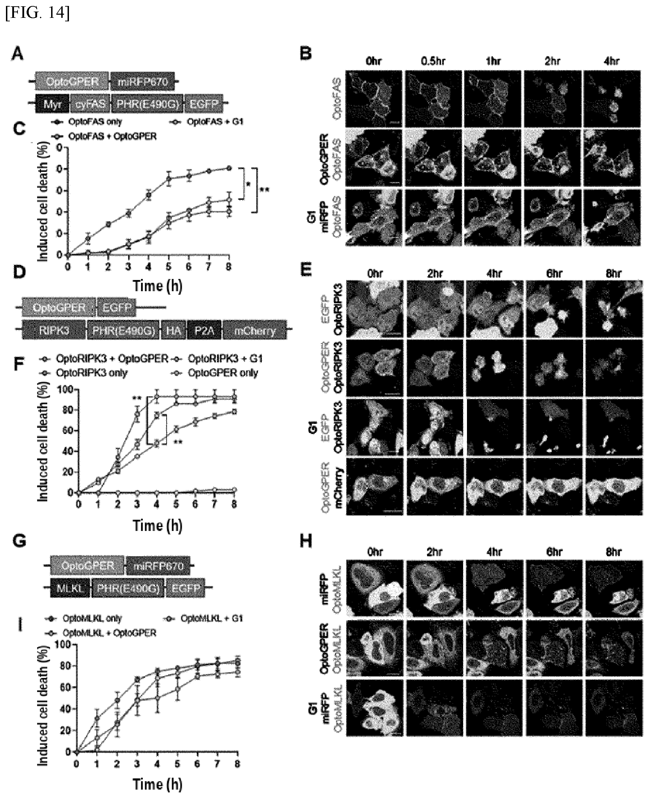

From the HeLa cell line experiments and TCGA database analyses, it was confirmed that activation of GPER induces expression of genes related to inhibition of apoptosis and increases expression of genes related to cellular responses according to TNF activation. Upon binding of the TNF ligand, cells may induce cell proliferation-related factors through NF-kB activation depending on conditions, whereas different forms of cell death, namely apoptosis and necroptosis, may also occur (Webster JD, Vucic D. Front Cell Dev Biol 2020). In order to confirm the effects of GPER activation on apoptosis and necroptosis pathways, respectively, experimental methods capable of specifically inducing apoptosis and necroptosis were required. Accordingly, the previously reported optogenetic apoptosis induction system OptoFAS (Kim et al., Sci Adv 2020) and optogenetic necrosis induction systems OptoRIPK3 and OptoMLKL (Shkarina et al., J Cell Biol 2022; Taslimi et al., Cell Death Discovery 2023) were co-transfected together with OptoGPER, and cell death patterns according to optogenetic activation were confirmed through real-time confocal imaging and quantitatively analyzed (FIG. 14).

Upon optogenetic activation of OptoFAS alone, apoptosis of approximately 80% of cancer cells was observed within 8 hours. In contrast, when optoGPER was activated together with optoFAS, a statistically significant decrease in cell death was confirmed (FIGS. 13A to 13C). This result is consistent with the activation of the negative apoptotic process observed in the RNA-seq analysis of optoGPER, and suggests that signaling pathway activation according to GPER activation affects the apoptosis pathway.

When receptor-interacting serine/threonine protein kinase 3 (RIPK3), which is a necrotic cell death pathway factor, was activated using optoRIPK3, cell death was observed. Interestingly, when OptoGPER was simultaneously activated, cell death was accelerated, and particularly, statistically significantly faster cell death was induced between 0 and 4 hours (FIGS. 13D to 13F).

In contrast, in the case of MLKL, which is also a necroptosis pathway factor and an immediate downstream pathway factor of RIPK3, no difference in the degree of induction of programmed necroptosis was observed between optoMLKL activation alone and simultaneous activation with optoGPER (FIGS. 13G to 13I). When G1, a ligand capable of activating GPER while inducing each type of cell death, was treated, results similar to those of optoGPER were observed. In summary, when cell death is induced, simultaneous activation of GPER suppresses the apoptosis pathway while accelerating the programmed necroptosis pathway, particularly involving the RIPK3 activation stage.

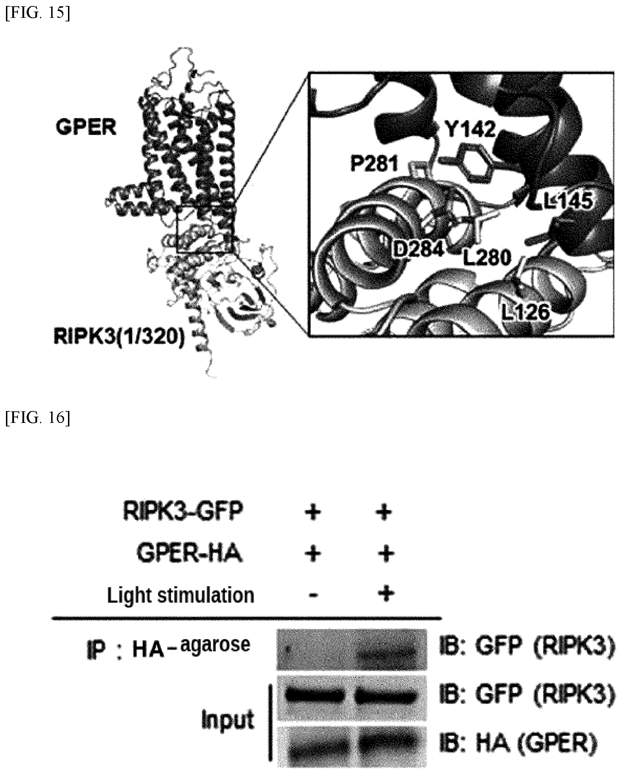

This can also be inferred from protein binding prediction results using Alphafold (https://colab.research.google.com/github/sokrypton/ColabFold/ blob/main/AlphaFold2.ipynb). Prediction of the protein interaction and structure between GPER and RIPK3 using PyMOL and pLDDT software showed that the C-terminal region of GPER is predicted to bind to RIPK3, and Y142 and L145 of GPER were identified as regions interacting with RIPK3 (FIG. 15).

In addition, direct binding between GPER and RIPK3 was confirmed through co-immunoprecipitation (FIG. 16). Through this, it can be inferred that direct structural binding between GPER and RIPK3 induces promotion of RIPK3 activation.

### 3-2. Detailed Protocol for Co-immunoprecipitation Experiment

CMV-optoGPER-HA and CMV-optoRIPK3-EGFP were transfected into HEK293T cells using Lipofectamine LTX reagent (Invitrogen, 15338-030) according to the manufacturer's instructions. At the same time, the medium was replaced with phenol red-free DMEM (Cat. LM 001-10, Wellgene) supplemented with 10% fetal bovine serum (FBS) stripped with dextran-coated charcoal (Sigma, C6241-5G), 1% penicillin and streptomycin, and maintained in a humidified atmosphere containing 5% CO₂ at 37°C. One day later, light stimulation was applied for 1 hour at 488 nm / 919.43 uW/cm². Cell lysates were prepared using IP lysis buffer (Thermo Fisher Scientific) together with a protease/phosphatase inhibitor cocktail (Cell Signaling Technology). For immunoprecipitation of hemagglutinin (HA)-conjugated GPER1 protein and interacting molecules thereof, equal amounts of proteins from the cell lysates were incubated with anti-HA agarose (Thermo Fisher Scientific) for 4 hours. After washing and elution, the purified proteins were subjected to western blot analysis using anti-GFP (Santa Cruz Biotechnology, Texas, USA) and anti-HA (Cell Signaling Technology).

### 3-3. Confirmation of Increased Activation of the Programmed Necroptosis Pathway According to Simultaneous Activation of GPER and RIPK3

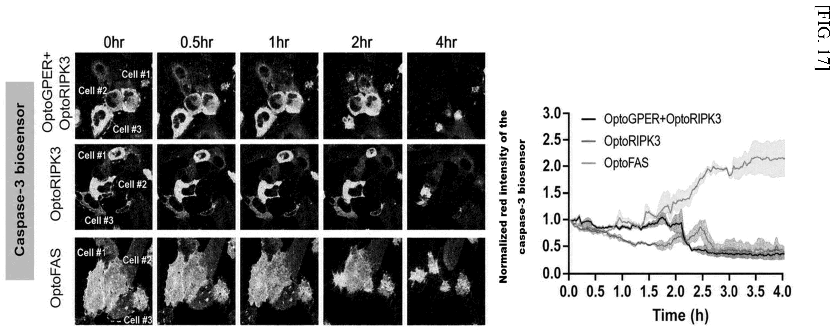

From the preceding results, it can be seen that simultaneous activation of GPER and RIPK3 induces rapid activation of RIPK3 through binding between GPER and RIPK3 and accelerates the programmed necroptosis pathway. First, it was confirmed that the accelerated cell death observed when optoGPER and optoRIPK3 were simultaneously activated was not apoptosis. The Caspase-3 biosensor is a FRET-based sensor that converts from green fluorescence to red fluorescence when intracellular caspase-3 acts thereon. When apoptosis was induced using OptoFAS, conversion to red fluorescence was detected as cells died. In contrast, when cell death was induced by activating optoRIPK3 alone or together with optoGPER, red fluorescence was not detected in the cells (FIG. 17).

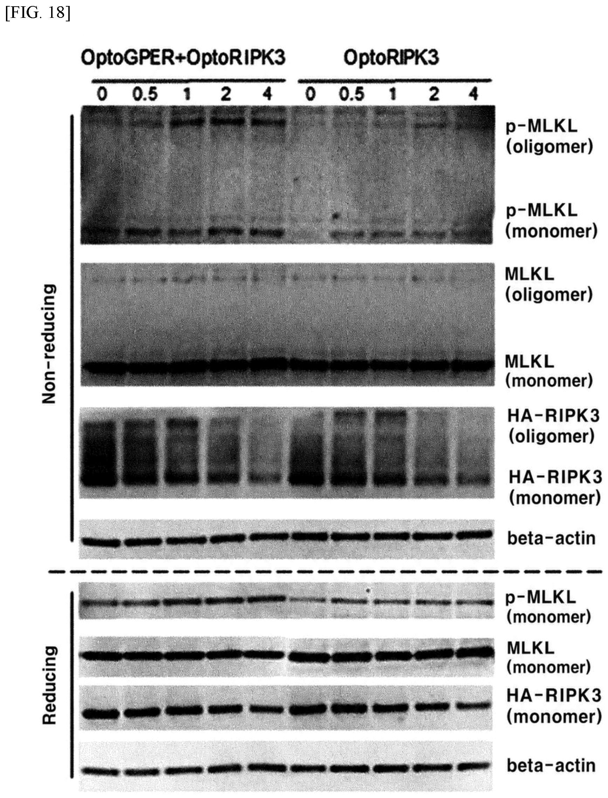

In addition, western blot experiments confirmed that phosphorylation of RIPK3 increased from 1 hour after activation when optoRIPK3 alone was induced or when optoGPER was simultaneously induced. Upon simultaneous activation of OptoGPER and optoRIPK3, MLKL, which is a downstream pathway factor of RIPK3, was activated, and p-MLKL increased more strongly at an earlier time point compared to activation of optoRIPK3 alone (FIG. 18).

### 3-4. Detailed Protocol for Western Blot Experiment

Cell lysates were obtained using cell lysis buffer (Cell Signaling Technology, Massachusetts, USA) together with a protease/phosphatase inhibitor cocktail (Cell Signaling Technology). Protein concentrations were evaluated using a Bradford protein assay kit (Thermo Fisher Scientific, Massachusetts, USA). Subsequently, equal amounts of proteins were separated using 4 to 12% Bis-Tris gradient gels (Thermo Fisher Scientific) together with MOPS running buffer (Thermo Fisher Scientific) according to the manufacturer's instructions. The separated proteins were transferred onto polyvinylidene difluoride membranes. After blocking with skim milk or 5% BSA, the membranes were incubated with appropriate primary antibodies and HRP-conjugated secondary antibodies according to the instructions provided by the manufacturer. After washing, the membranes were imaged using chemiluminescence (iBright CL1000, Thermo Fisher Scientific). The antibodies used are as follows: anti-pMLKL (Abcam), anti-MLKL (Cell Signaling Technology), anti-HA (Cell Signaling Technology), anti-AKT (Cell Signaling Technology), anti-ERK (Cell Signaling Technology), anti-pAKT(S473) (Cell Signaling Technology), anti-pERK(T202/Y204) (Cell Signaling Technology), and anti-beta actin (GeneTex).

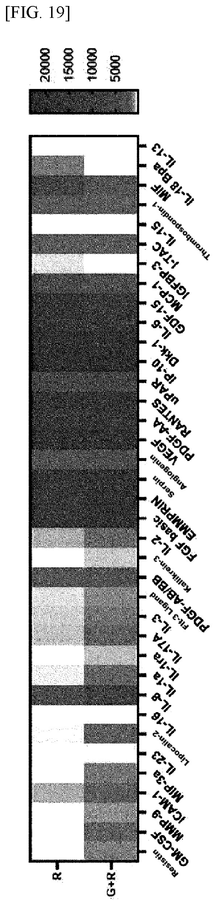

When the programmed necroptosis pathway is activated and cellular damage occurs, substances such as cytokine types called DAMPs (damage-associated molecular patterns) are secreted extracellularly. When a cytokine array was performed, it was confirmed that, compared to induction of optoRIPK3 alone (R), simultaneous activation of optoGPER (G+R) resulted in secretion of relatively more cytokines, with IL-8 being representatively activated (FIG. 19).

### 3-5. Detailed Experimental Protocol for Cytokine Array Experiment

To remove debris and cells, cell supernatants were centrifuged after 4 hours of light stimulation and filtered through a 0.25 µm syringe filter. Cytokine arrays were analyzed using a Proteome Profiler Human XL Cytokine Assay Kit (R&D Systems, ARY022B) according to the manufacturer's instructions. Quantification of spots was performed using the ImageJ plugin Protein Array Analyser.

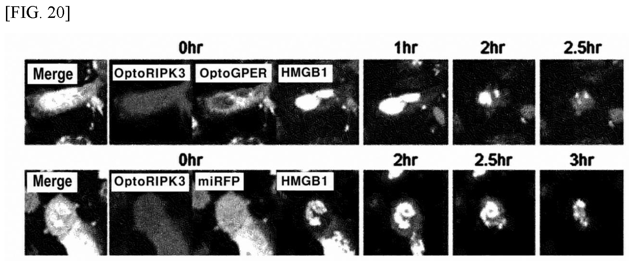

In addition, as one of the DAMPs, the HMGB1 protein located in the nucleus is translocated to the cytoplasm and secreted extracellularly during cell death. When real-time confocal imaging was performed using a construct in which GFP was tagged to HMGB1 to visually confirm intracellular translocation, it was likewise confirmed that simultaneous activation of optoRIPK3 and optoGPER accelerated translocation of HMGB1 from the nucleus to the cytoplasm (FIG. 20).

Through the above experimental results, it was confirmed that when activation of GPER is added to the programmed necroptosis pathway initiated from activation of RIPK3, acceleration of programmed necroptosis pathway activation occurs.

### 4. Integrated Transcriptomic Analysis of the Effects of GPER on RIPK3 Activation

### 4-1. RNA-seq Analysis

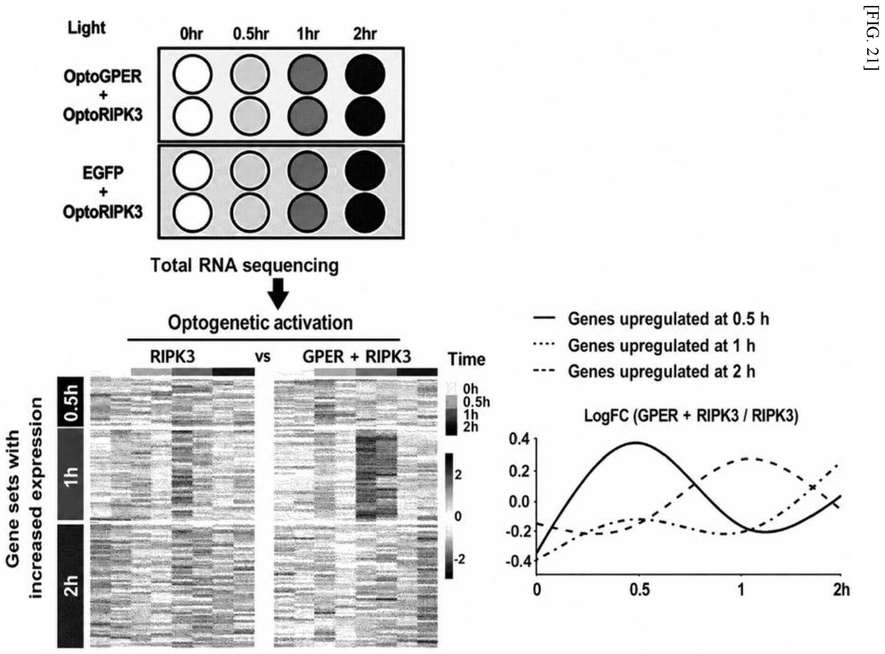

In order to more comprehensively analyze the effects of GPER on RIPK3 activation, transcriptome analysis through mRNA expression changes was performed using total RNA-seq under conditions of activation of optoRIPK3 alone and simultaneous activation of optoRIPK3 and optoGPER. Compared to activation of optoRIPK3 alone, simultaneous activation of optoGPER and optoRIPK3 showed considerably different patterns of gene expression over time (0.5, 1, and 2 hours) (FIG. 21). The heatmap represents enriched gene levels for genes significantly increased upon combined activation (GPER/RIPK3) compared to optoRIPK3 activation at each indicated time point (p-value < 0.05 and logFC > 0.2). logFC and p-values were calculated after adjusting gene expression levels at the indicated time points relative to baseline (0 hour) expression levels. RNA-seq libraries were prepared using TruSeq stranded RNA (Illumina) as in the previous experiments, and NovaSeq6000 Sequencing (100 bp paired-end, 6 Gb) was performed (Macrogen).

### 4-2. GO Analysis

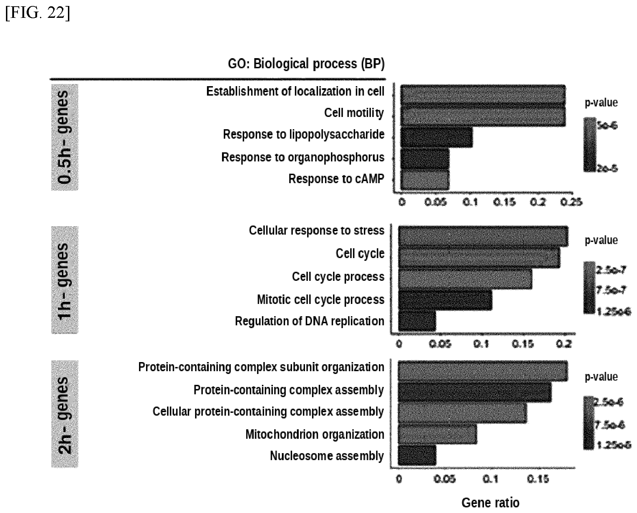

When biological process (BP) analysis among GO analyses was performed on differentially expressed genes (DEGs) showing different expression upon simultaneous activation of optoRIPK3 and optoGPER compared to activation of optoRIPK3 alone, genes related to factors necessary for increased intracellular activity, such as response to organophosphorus and response to cAMP, were increased at 0.5 hours. In addition, at 1 hour, expression of genes related to cellular response to stress increased, from which activation of the programmed necroptosis pathway could be inferred. In relation thereto, changes in expression of genes related to cell proliferation, such as cell cycle process and DNA replication, were also observed. Upon activation for 2 hours, increases in activation of processes related to protein-level changes, such as protein assembly, and factors related to the mitochondrial pathway included in the downstream programmed necroptosis pathway were also confirmed (FIG. 22).

### 4-3. GSEA Analysis

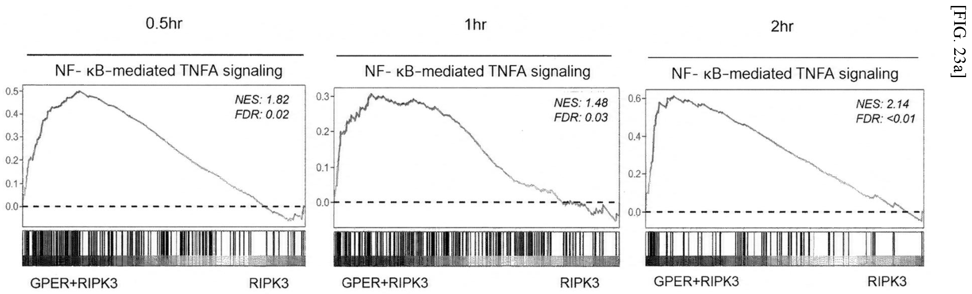

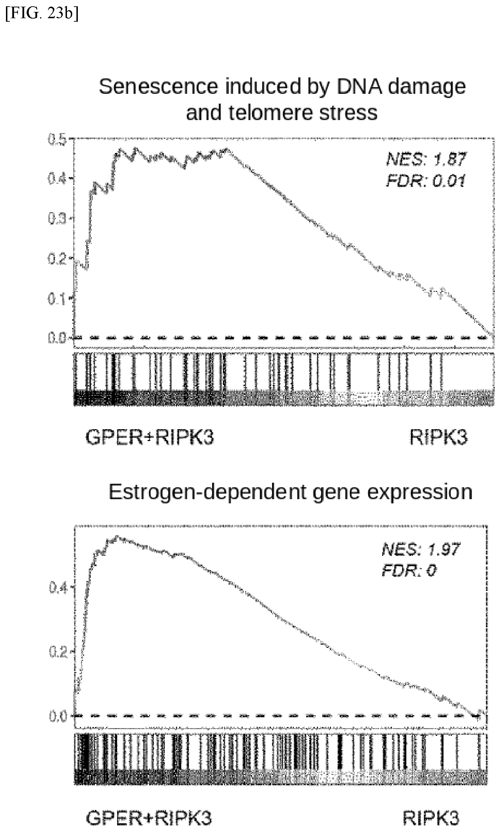

In addition, when GSEA analysis was performed using HALLMARK and REACTOME gene sets, TNFa signaling was statistically significantly increased at 30 minutes and 1 hour when optoGPER was simultaneously activated compared to samples in which only optoRIPK3 was activated, and increases in diseases of programmed cell death and estrogen dependent gene expression were confirmed at 2 hours (FIG. 23).

### 4-4. Activated Pathway Factor Map in the Programmed Necrosis KEGG Pathway

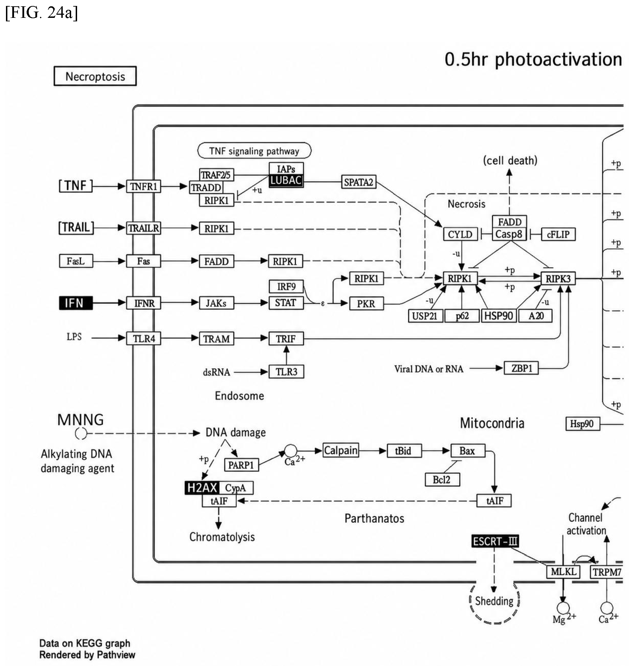

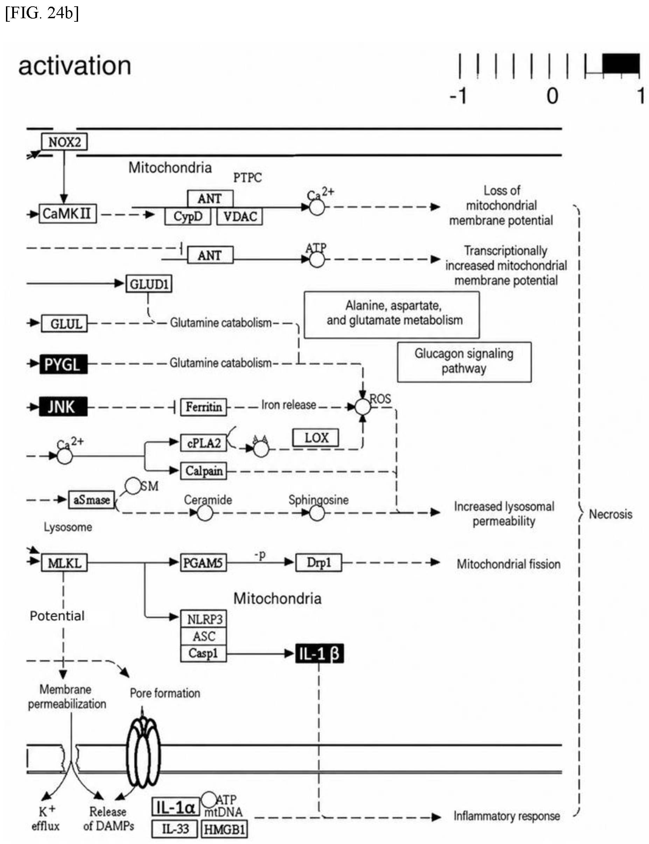

When RNA-seq results obtained after simultaneous activation of optoRIPK3 and optoGPER were applied to various factors in the programmed necroptosis KEGG pathway map, relative activation of various programmed necroptosis pathway factors was observed at multiple time points (0.5, 1, and 2 hours) (FIG. 24).

Through the above results, it can be concluded that activation of GPER interacts with RIPK3 in cells in which the programmed necroptosis pathway has been initiated, thereby increasing expression of various programmed necroptosis pathway factors and inducing rapid programmed necroptotic cell death.

The present invention was carried out through the following projects.

[National Research and Development Project Supporting the Present Invention]
[Project Unique Number] 1711191473
[Project Number] 2021R1A4A1031856
[Ministry] Ministry of Science and ICT
[Project Management Organization] National Research Foundation of Korea
[Research Program Name] Group Research Support
[Research Project Name] Immunogenic Cell Death Program Laboratory
[Contribution Rate] 1/2
[Research Performing Organization] Ajou University
[Research Period] 2021.06.01 ~ 2024.02.29
[National Research and Development Project Supporting the Present Invention]
[Project Unique Number] 1711192406
[Project Number] 2022R1C1C1007289
[Ministry] Ministry of Science and ICT
[Project Management Organization] National Research Foundation of Korea
[Research Program Name] Basic Individual Research (MSIT)
[Research Project Name] In Patient-Derived Complex Pancreatic Cancer Organoid Models
Through Optogenetic Steroid Receptor Activation Control
Analysis of Tumor Microenvironment Signaling and Discovery of Therapeutic Targets
[Contribution Rate] 1/2
[Research Performing Organization] Ajou University
[Research Period] 2022.03.01 ~ 2025.02.28

## Claims

1. A fusion protein optogenetically activated by blue light, comprising:
an extracellular domain of rhodopsin;
a transmembrane domain of rhodopsin; and
an intracellular domain of a G-protein coupled estrogen receptor (GPER) bound to the transmembrane domain.

2. The fusion protein of claim 1, wherein the fusion protein is encoded by the nucleotide sequence of SEQ ID NO: 1.

3. The fusion protein of claim 1, wherein the fusion protein is activated by irradiation with blue light having a wavelength of 350 to 500 nm and an intensity of 500 to 1500 Uw/cm² for 1 to 10 days.

4. The fusion protein of claim 1, wherein the fusion protein increases mRNA expression of one or more genes selected from the group consisting of c-FOS, PTGS2, CTGF, EGR1, MMP2, and COL12A1.

5. The fusion protein of claim 1, wherein the fusion protein phosphorylates extracellular signal-regulated kinase (ERK).

6. The fusion protein of claim 1, wherein the fusion protein inhibits apoptosis and induces programmed necroptosis.

7. The fusion protein of claim 6, wherein the programmed necroptosis is mediated through binding to RIPK3.

8. A polynucleotide encoding the fusion protein according to claim 1.

9. A recombinant vector comprising the polynucleotide of claim 8.

10. The recombinant vector of claim 9, wherein the vector is one or more viruses selected from the group consisting of adeno-associated virus (AAV), adenovirus, retrovirus, lentivirus, modified vaccinia virus Ankara (MVA), herpes simplex virus, and baculovirus.

11. A method for preparing a fusion protein activated by blue light, comprising:
treating a cell with the vector of claim 9; and
irradiating the cell with blue light to induce photoactivation.

12. The method of claim 11, wherein the fusion protein is activated by irradiation with blue light having a wavelength of 350 to 500 nm and an intensity of 500 to 1500 µW/cm² for 1 to 10 days.

13. A composition for regulating an activity of a fusion protein by blue light irradiation, comprising the fusion protein of claim 1 or a polynucleotide encoding the same.

14. The composition of claim 13, wherein the composition inhibits apoptosis and induces programmed necroptosis.
